# EUROPEAN PATENT SPECIFICATION

(11) **EP 0 454 645 B1**
(45) Date of publication and mention of the grant of the patent: **23.11.1994**
(21) Application number: 91850102.4
(22) Date of filing: 23.04.1991
(51) Int. Cl.: A61F 2/42, A61F 2/30

(54) **Artificial joint mechanism**
Künstlicher Gelenkmechanismus
Mécanisme d'articulation artificielle

(30) Priority: 23.04.1990 SE 9001430
(43) Date of publication of application: 30.10.1991
(73) Proprietor: MEDEVELOP AB, S-431 69 Mölndal (SE)
(72) Inventor: Branemark, Per-Ingvar, S-431 69 Mölndal (SE)
(74) Representative: Westerlund, Christer John Axel

(56) References cited:
- EP-A- 0 057 597
- DE-A- 1 960 087
- FR-A- 2 244 445
- GB-A- 2 169 512
- US-A- 3 955 282
- US-A- 3 990 116
- US-A- 4 313 232

## Description

The present invention relates to an artificial, elastic joint mechanism intended for use in reconstructing joints such as finger joints, wrists, ankles, elbows, hip-joints, knees, toe joints and other joints in the human body. The joint mechanism can also be used to replace disc functions in the spine.

A joint body for joint prostheses consisting entirely of an elastomeric material is described, for instance, in US Patent 3,875,594. This known body is provided with pins protruding from its opposing short sides and formed in one piece with the body, said pins being designed to fit into cavities in respective bone pipes. This direct anchoring of the joint body in the bone tissue has been found to give rise to serious drawbacks, such as undesired wear between the pins and the surrounding tissue upon movement of the joint, with consequent risk of inflammation and tissue damage.

To eliminate the latter drawback, according to Swedish patent Application No. 87 04 211-5 corresponding to US Application No. 473,992, filed May 2, 1990 a combination is suggested comprising a mid section of resilient material arranged as a joint body between a pair of pins, the pins being attached by means of flanges embedded in the material and arranged transversely to the pins, and a tubular screw for each pin which can be secured in the bone, into which the pin is inserted, the pins and screws consisting of a biocompatible material. This construction has enabled better anchoring of the joint prosthesis in the bone pipes than was possible in the construction according to US Patent 3,875,594 mentioned above.

However, it has been found that the joint body used in both the above-mentioned constructions, which consists of silicon rubber or polyurethane, is not satisfactory with respect to the expectations which must be placed on such an artificial joint. First of all, there is considerable risk at tissue damage and inflammation due to the constant movement of the pins in the tissue or in the attachment member. Furthermore, it has been found in practice that the joint body itself is not sufficiently strong in the environment in which it is used. A certain bending resistance is desired in such joint bodies, i.e. a more or less directed bending function, in order to emulate the normal function of a joint as much as possible.

Also known in the art (DE-A-1 960 087) is an artificial joint mechanism for replacing a natural joint and comprising a joint body which includes a flexible mid-section having a central constriction, rotationally symmetrical tapering connecting means integral with the joint body for connecting it to bone tissue, and reinforcement means in at least said flexible mid-section for optimizing flexibility in the main bending direction of the joint mechanism and or the pivotability of the joint mechanism.

WO-A-8903663 discloses pins protruding from the flexible mid-section of an artificial joint mechanism, said pins being designed for cooperation with anchoring means implanted in the bone tissue on each side of the joint.

An object of the present invention, therefore, is to achieve a joint body which is more durable in use, and which can be optimally adjusted to the bending function of the joint it is replacing.

This and other objects are achieved by providing an artificial joint mechanism for replacing a natural joint and comprising a joint body including a flexible mid-section, connecting means for connecting said joint body to bone tissue, and reinforcement means in at least said flexible mid-section for optimizing flexibility of said mid section in the main bending direction of the joint mechanism and/or the pivotability of the joint mechanism, wherein the connecting means comprises a pair of pins connected to said flexible mid-section and protruding therefrom for cooperation with anchoring members inplanted in the bone tissue on each side of the joint, said joint body having the general form of a hemisphere, one of said connecting means protruding from its flat side and another protruding from its rounded side, said reinforcement means comprising a wire in the joint body, said wire being helically coiled threrein to form a coil, the helix of which extends towards said connecting means.

According to a suitable embodiment of the invention, the mid-section with protruding pins, consists of a thermoplast containing di-phenyl-sulphone groups of the type 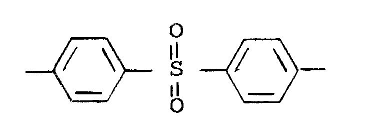
The material proposed, according to the invention, has the ability to return to its original form to at least 80 % after repeated, varying deformations. That means that it will not return 100% to its origianl shape but nearly to that shape, i.e. about at least 80 %.

The joint mechanism described herein includes reinforcement to optimize flexibility in the main bending direction of the reconstructed joint and/or pivotability of the joint mechanism.

Other features and advantages of the present invention will become apparent from the following description of the invention which refers to Figures 13 and 14 of the accompanying drawings. Figures 1 to 12 show some artificial joints which do not fall within the scope of protection of Claim 1.
Figure 1 shows a section through a reinforced joint body;
Figure 2 shows a section throuch another embodiment of a joint body; and
Figures 3-5 show additional alternate embodiments, Figure 4 in section, and Figures 3 and 5 in perspective views.
Figures 6-9 show sections through further embodiments of a joint body with the "a" Figures showing transverse sections and the "b" Figures showing top view cross sections.
Figure 10 is a perspective of a further embodiment of a joint body.
Figure 11 is a top view cross section through yet another embodiment.
Figure 12 is a top view cross section through a still further embodiment.
Figure 13 is a top view of an embodiment of a joint body according to the invention.
Figure 14 is a side view thereof.

Referring to the drawings, a joint body is designated 1. When the joint body 1 is used together with anchoring elements screwed into respective bones, the joint body 1 is provided with a pair of protruding pins 2 intended to cooperate with cavities arranged in the anchoring elements.

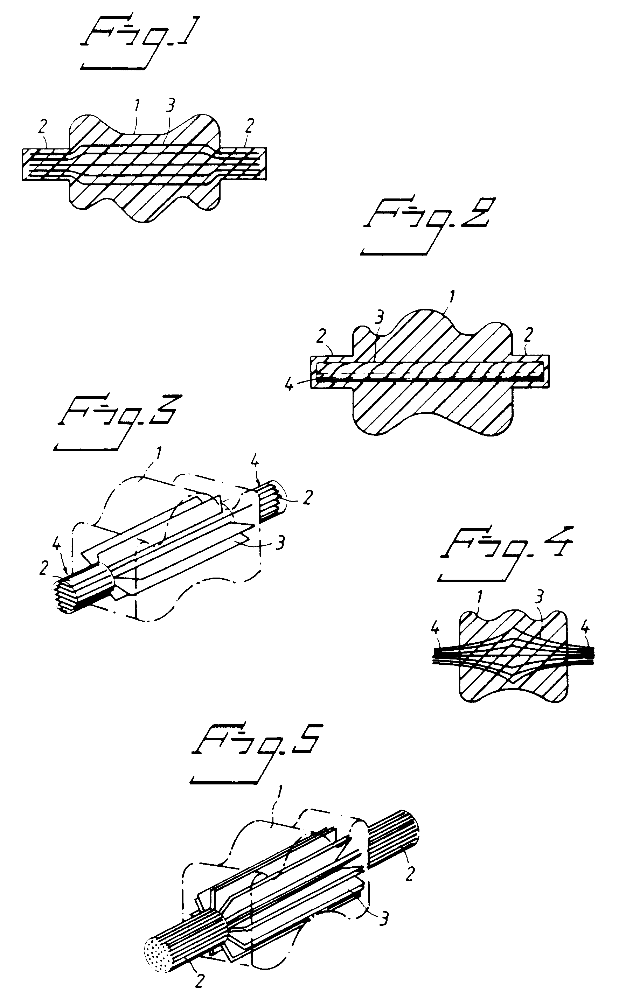

The joint body 1 preferably comprises an elastomeric material, and in the embodiment according to Fig. 1 it is provided with reinforcement means 3 in the form of a number of fibers extending longitudinally through the mid-section between pins 2 and out into the pins.

Reinforcement means 3 may consist, for instance, of titanium threads, carbon fibers, polymer fibers, cable-like twisted fibers, mesh of plastic or metal, suitably titanium, wowen inserts of biocompatible material, thin strips, or thin plates, of primarily biocompatible material, preferably titanium or the like.

In the embodiment according to Fig. 2, reinforcement means 3 consists of a wire 4 of titanium. Here, also, reinforcement means 3 suitably extends out into the pins 2.

In the embodiments according to Figs. 1 and 2, reinformeans 3 has been embedded into the joint body 1 and the pins 2 by means of injection molding, so that the elastomeric material surrounds the reinforcement of the actual pins 2, as well.

In the embodiment according to Figs. 3, 4 and 5, the reinforcement 3 has a special configuration in which the end portions 4 which are to form the pins 2 have been compressed and are, therefore, not necessarily embedded in the elastomeric material.

Figs. 6-12 show some principles as to the structure of the reinforcement 3.

According to Figs. 6a and b, thus, the reinforcement 3 may consist of a number of thin strips 5, or plates 5, of titanium foil, for instance, arranged spaced from each other, their narrowing end portions 6 and 7 extending into the pins 2.

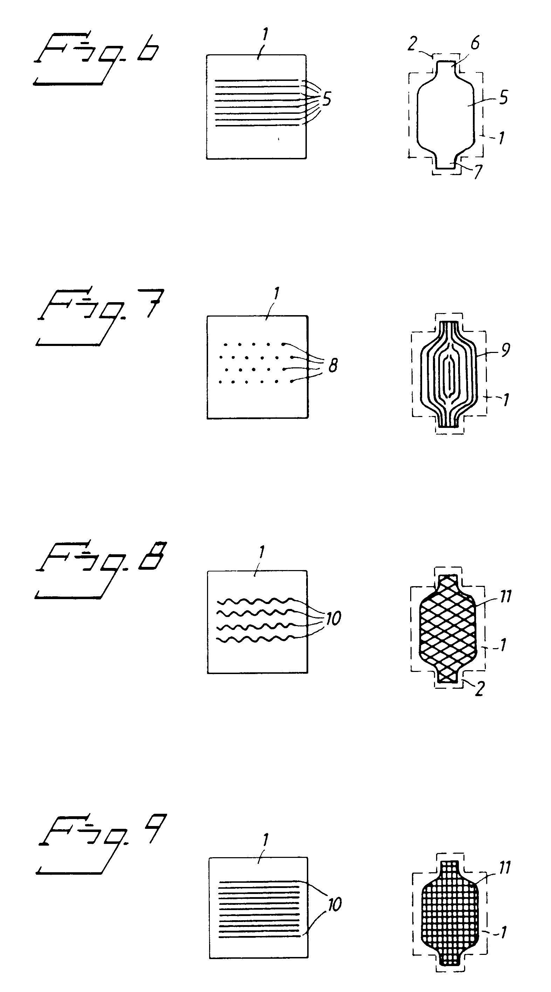

According to Figs. 7a and b, the reinforcement 3 consists of a number of layers 8 of thin threads 9, the ends of which continue into the pins 2.

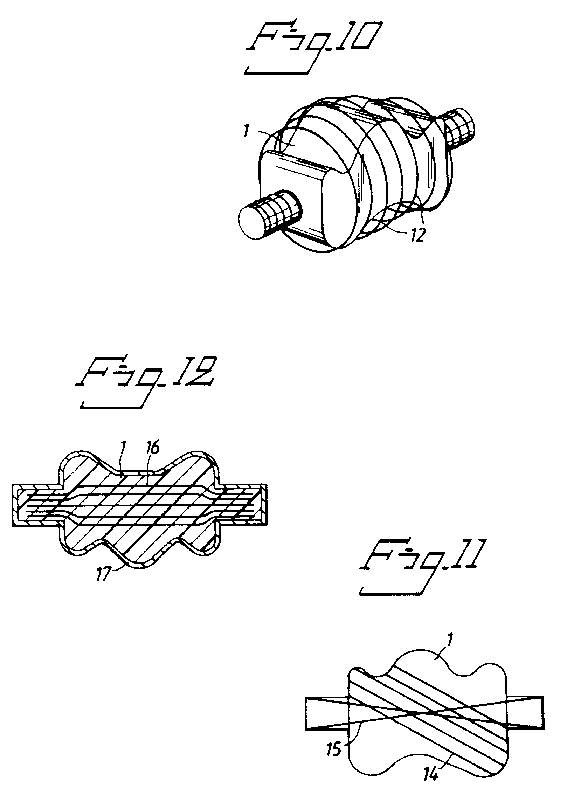

According to Figs. 8a and b, the reinforcement 3 consists of one or more layers 10 of a net-like insert 11.

According to Figs. 9a and b, one or more layers 10 of a woven material 11 are used to form the reinforcement 3.

The flexibility and elasticity of joint bodies 1, reinforced in such manner, can be governed as to direction and strength, depending on where the reinforcement is placed in the section and in which plane it is spread.

Depending on the desired degree of flexibility, control, etc., the reinforcement may also be given various three-dimensional embodiments. For instance, according to Fig. 10, the reinforcement may be arranged spirally around the joint body, i.e. it is a fiber 12 wrapped spirally around the pins at the ends of the body and around the body itself. The reinforcement may be on the joint body in some other suitable manner in order to achieve the desired optimization with respect to limited flexibility, compression, etc.

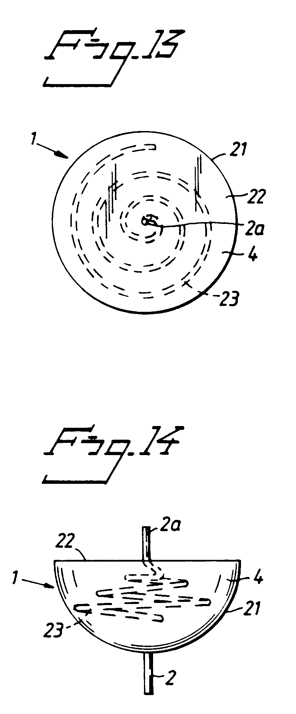

The reinforcement can also be applied outside the actual joint body in the form of a spiral, as in Fig. 10, or the like. The fibers may run diagonally in the joint body itself as with the fibers 14, 15 in Fig. 11. Some fibers 14 are in the body and some fibers 15 extend from pin to pin.

As in Fig. 12, the entire joint body 1 with the reinforcement 16 located either outside or inside the body itself can be surrounded by a biocompatible sleeve 17 of titanium, for instance, suitably with slight clearance, so that the surrounding tissue is not affected directly by bending movements of the joint body.

In the embodiment of the joint body according to the invention of Figs. 13 and 14, the joint body 1 has a generally half spherical shell 21 including a rounded body with a flat end wall 22. The shell surrounds and defines the shape of the elastomeric material body. The pin 2 at one end is integral with and of the material of the body 1. The pin 2a at the other end of the joint body projects out from the flat end wall 22 and is an integral end portion of the internal reinforcement 23, which is in the form of a helical coil which decreases in cross section toward the pin 2a. This embodiment has a special advantage in a joint. It permits a certain degree of rotation of the joint body with reference to the adjacent bone, to the extent permitted by the pins 2.

According to a preferred embodiment of the invention, the elastomeric material used for the joint body 1 may be a thermoplastic containing di-phenyl-sulphone groups, characterized by good elasticity, high durability and notch impact strength. In the present example, a poly-sulphonic plastic marketed by Amoco Performance Products, Inc. under the trade name UDELL, designation P1700, is used. This poly-sulphonic plastic is produced by a nucleophillic substitution reaction between the di-sodium salt of Bisphenol A and 4,4-di-chlor-di-phenyl sulphone. The chemical structure then has the following appearance: 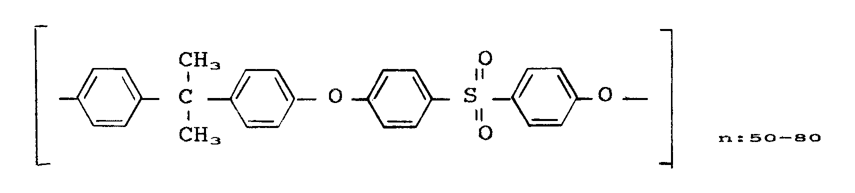
The most important consituent is the di-phenyl sulphone group 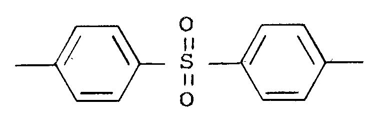
Ether and the isopropylide have been connected to the diphenyl-sulphone group in order to give the material elasticity, as well as high strength. The material properties are as follows:

| | |
|---|---|
| Tensile strength | 70 N/mm² |
| Tension modulus | 2480 N/mm² |
| Impact strength | 40 Kj/m² |

Preferred tensile strength is between 50 and 90 N/mm². Preferred modulus of elasticity is between 2000 and 2600 N/mm². Preferred notch impact strength is between 40 and 80 Kj/m² (with a material thickness of 2.8-15.0 mm).

It should also be pointed out here that the polysulphonic plastic is inert with respect to the tissue fluid and is substantially impervious to water. The ability of the material to regain its original form after repeated, varying deformations is about at least 80%, that is, it does not fully restore to its original shape by itself. The notch impact strength stated above was measured with material thickness of between 2.8 and 15.0 mm.

The invention is, of course, not limited to the embodiments described above and the reinfrocements can be effected in many ways in order to provide optimal joint function for every occasion. Although the present invention has been described in relation to particular embodiments thereof, many other variations and modifications and other uses will become apparent to those skilled in the art. It is preferred, therefore, that the present invention be limited not by the specific disclosures herein, but only by the appended claims.

## Claims

1. An artificial joint mechanism for replacing a natural joint and comprising a joint body (1) including a flexible mid-section connecting means (2) for connecting said joint body (1) to bone tissue, and reinforcement means (3) in at least said flexible mid-section for optimizing flexibility of said mid section in the main bending direction of the joint mechanism and/or the pivotability of the joint mechanism, said connecting means comprising a pair of pins connected to said flexible mid-section and protruding therefrom, **characterized** in that said pins are arranged for cooperation with anchoring members implanted in the bone tissue on each side of the joint, said joint body (1) having the general form of a hemisphere (21), one of said connecting means (2a) protruding from its flat side and another (2) protruding from its rounded side, said reinforcement means (3) comprising a wire in the joint body (1), said wire being helically coiled threrein to form a coil (23), the helix of which extends towards said connecting means (2, 2a).

2. The artificial joint mechanism of claim 1, **characterized** in that the diameter of the helical coil (23) in the joint body (1) diminishes in the direction from the rounded side towards the flat side.

3. The artificial joint mechanism of claim 1, **characterized** in that the connecting means at the flat side of the joint body (1) is a pin (2a) integral with the coil (23).

4. The artificial joint mechanism of claim 1 to 3, **chracterized** in that the reinforcement means (3) is shaped and comprised by a material for resiliently maintaining the joint body (1) in its original shape following repeated deformation of said body (1) in its main bending direction.

5. The artificial joint mechanism of claim 1 to 4, **characterized** in that said reinforcement means (3) comprises at least one reinforcement element extending along said joint body (1), said element being selected from the group consisting of titanium threads, carbon fibres, polymer fibres, cable-like twisted fibres, plastic or metal mesh, woven inserts comprising biocompatible material, and thin strips or plates comprising biocompatible material.

## Patentansprüche

1. Künstlicher Gelenkmechanismus als Ersatz für ein natürliches Gelenk, bestehend aus einem Gelenkkörper (1) mit einem biegsamen Mittelteil, Anschlußelementen (2) für die Verbindung des besagten Gelenkkörpers (1) mit dem Knochengewebe, und Verstärkungen (3) in mindestens dem biegsamen Mittelteil zur Optimierung der Biegsamkeit des besagten Mittelteils in der Hauptbiegerichtung des Gelenkmechanismus und/oder der Drehrichtung des Gelenkmechanismus; wobei die besagten Anschlußelemente aus zwei Stiften bestehen, die mit dem besagten biegsamen Mittelteil verbunden sind und in entgegengesetzten Richtungen von diesem Teil abstehen; **dadurch gekennzeichnet,** daß die besagten Stifte so geformt sind, daß sie genau in Verankerungselemente passen, die im Knochengewebe auf beiden Seiten des Gelenks implantiert werden; wobei der besagte Gelenkkörper (1) generell die Form einer Halbkugel (21) hat, wobei eines der Verbindungselemente (2a) aus der flachen Seite der Halbkugel und das andere Element (2) aus der gewölbten Seite der Halbkugel ragt; wobei die besagte Verstärkung (3) des Gelenkkörpers (1) aus einem wendelförmig angeordneten Draht (23) besteht, wobei die Wendel bis in die besagten Verbindungselemente (2,2a) reicht.

2. Künstlicher Gelenkmechanismus nach Anspruch 1, **dadurch gekennzeichnet,** daß der Durchmesser der Wendel (23) im Gelenkkörper (1) zwischen der gewölbten Seite der Halbkugel und der flachen Seite kontinuierlich abnimmt.

3. Künstlicher Gelenkmechanismus nach Anspruch 1, **dadurch gekennzeichnet,** daß das Verbindungselement an der flachen Seite des Gelenkkörpers (1) ein Stift (2a) ist, der fest mit der Wendel (23) verbunden ist.

4. Künstlicher Gelenkmechanismus nach Anspruch 1 bis 3, **dadurch gekennzeichnet,** daß die Verstärkung (3) von der Form und vom Material her so ausgelegt ist, daß der Gelenkkörper (1) eine elastische Verformung erfährt und der Gelenkkörper (1) auch nach wiederholten Biegebeanspruchungen in der Hauptbiegerichtung wieder in seine ursprüngliche Form zurückkehrt.

5. Künstlicher Gelenkmechanismus nach Anspruch 1 bis 4, **dadurch gekennzeichnet,** daß die besagte Verstärkung (3) mindestens ein Verstärkungselement enthält, das in Längsrichtung des Gelenkkörpers (1) verläuft, wobei das besagte Element bestehen kann aus Titanfäden, Carbonfasern, Polymerfasern, verdrillten Fasern, Kunststoff- oder Metallgittern, gewebten Einlagen aus körperverträglichem Material und dünnen Streifen oder Platten aus körperverträglichem Material.

## Revendications

1. Mécanisme d'articulation artificielle destiné à remplacer une articulation naturelle et comportant un corps d'articulation (1) comprenant une section médiane flexible, des moyens de raccordement (2) destinés à raccorder ledit corps d'articulation (1) à des tissus osseux, et des moyens de renfort (3) au moins dans ladite section médiane afin d'optimiser la flexibilité de ladite section médiane dans la direction de pliage principale du mécanisme d'articulation et/ou la capacité de pivotement du mécanisme d'articulation, lesdits moyens de raccordement comprenant une paire d'axes reliés à ladite section médiane et dépassant de celle-ci, caractérisé en ce que lesdits axes sont prévus pour coopérer avec des moyens d'ancrage implantés dans les tissus osseux de chaque côté de l'articulation, ledit corps d'articulation (1) ayant la forme générale d'un hémisphère (21), un desdits moyens de raccordement (2a) dépassant de son côté plat et l'autre dépassant de son côté arrondi, lesdits moyens de renfort (3) comportant un fil dans le corps d'articulation (1), ledit fil étant enroulé en hélice afin de former un enroulement hélicoïdal (23) dont l'hélice s'étend en direction lesdits moyens de raccordement (2, 2a)

2. Mécanisme d'articulation selon la revendication 1, caractérisé en ce que le diamètre de l'enroulement hélicoïdal (23) dans le corps d'articulation (1) diminue dans la direction allant du côté arrondi vers le côté plat.

3. Mécanisme d'articulation selon la revendication 1, caractérisé en ce que les moyens de raccordement au niveau du côté plat du corps d'articulation (1) sont constitués par un axe (2a) d'un seul tenant avec l'enroulement hélicoïdal (23).

4. Mécanisme d'articulation selon les revendications 1 à 3, caractérisé en ce que les moyens de renfort (3) sont mis en forme et sont constitués par une matière destinée à maintenir élastiquement le corps d'articulation (1) dans sa forme d'origine à la suite d'une déformation répétée dudit corps (1) dans sa direction de pliage principale.

5. Mécanisme d'articulation selon les revendications 1 à 4, caractérisé en ce que lesdits moyens de renfort (3) comportent au moins un élément de renfort s'étendant le long dudit corps d'articulation (1), ledit élément étant choisi dans le groupe constitué par les fils de titane, les fibres de carbone, les fibres polymères, les fibres torsadées en forme de câble, un treillis en matière plastique ou métallique, des inserts tissés comportant une matière biocompatible, et des bandes ou des plaques minces comportant une matière biocompatible.
